# EUROPEAN PATENT SPECIFICATION

(11) **EP 4 213 828 B1**
(45) Date of publication and mention of the grant of the patent: **27.08.2025**
(21) Application number: 21782647.8
(22) Date of filing: 13.09.2021
(51) Int. Cl.: A61K 31/19, A61K 31/661, A61K 31/7072, A61K 33/06, A61P 3/04, A61P 3/08, A61P 9/00, A61P 25/00, A61P 43/00

(54) **PHARMACEUTICAL COMPOSITION COMPRISING URIDINE AND PYRUVATE FOR REGULATING THE PROLIFERATION, ACTIVITY AND SURVIVAL OF IMMUNE CELLS**
PHARMAZEUTISCHE ZUSAMMENSETZUNG MIT URIDIN UND PYRUVAT ZUR REGULIERUNG DER PROLIFERATION, AKTIVITÄT UND DES ÜBERLEBENS VON IMMUNZELLEN
COMPOSITION PHARMACEUTIQUE COMPRENANT DE L'URIDINE ET DU PYRUVATE POUR RÉGULER LA PROLIFÉRATION, L'ACTIVITÉ ET LA SURVIE DES CELLULES IMMUNITAIRES

(30) Priority: 16.09.2020 EP 20196388
(43) Date of publication of application: 26.07.2023
(73) Proprietor: GV Squared S.r.l., 36100 Vicenza (IT)
(72) Inventor: VILLANI, Gaetano, 70124 Bari (IT); BATTAGLIA, Michele, 70124 BARI (BA) (IT)
(74) Representative: Schüssler, Andrea
(86) International application number: PCT/EP2021/075065
(87) International publication number: WO 2022/058271

(56) References cited:
- WO-A1-00/11952
- WO-A1-00/50043
- US-A1- 2002 049 182
- US-A1- 2004 224 920
- WEST A. PHILLIP ET AL: "Mitochondria in innate immune responses", NATURE REVIEWS IMMUNOLOGY, vol. 11, no. 6, 20 May 2011 (2011-05-20), London, pages 389 - 402, XP055785984, ISSN: 1474-1733, Retrieved from the Internet <URL:http://www.nature.com/articles/nri2975> DOI: 10.1038/nri2975
- ANGAJALA ANUSHA ET AL: "Diverse Roles of Mitochondria in Immune Responses: Novel Insights Into Immuno-Metabolism", FRONTIERS IN IMMUNOLOGY, vol. 9, 1 January 2018 (2018-01-01), pages 1605, XP055873112, Retrieved from the Internet <URL:https://www.ncbi.nlm.nih.gov/pmc/articles/PMC6052888/pdf/fimmu-09-01605.pdf> DOI: 10.3389/fimmu.2018.01605

## Description

The present invention concerns a composition comprising uridine and pyruvate for use in a method of regulating the proliferation, activity and survival of immune cells to ameliorate and/or prevent immunodeficiencies caused by impaired cellular respiration.

### Background of the Invention

Lack of immune system cells or impairment in the differentiation/activity of immune cells is found in many genetic, acquired or chronic diseases and ageing in general. During the last few years it was found that mitochondria play a keyrole in the etiology of these pathophysiological conditions and have a strong influence on the immunity as well (Angajala et al., Frontiers in Immunology, 2018, Vol. 9, Article 1605).

Mitochondria are semi-autonomous organelles possessing a replicating circular DNA (mtDNA) that encodes 13 protein subunits of the oxidative phosphorylation (OXPHOS) complexes, plus 22 transfer RNAs (tRNAs) and two ribosomal RNAs (12S and 16S rRNAs) needed for their protein synthesis machinery. Due to the endosymbiotic origin of mitochondria, organellar proteins, ribosomes and DNA share a close evolutionary proximity with their bacterial counterparts.

Several medicaments (e.g. antibiotics) can impair proper mitochondrial function. In particular, those medicaments can exert an off-target action on mitochondria causing a cellular metabolic shift towards a rho0-like phenotype, typical of mitochondrial DNA-depleted cells (WO 2015/014470 A1).

Mitochondria have the main task of generating ATP through OXPHOS; hence, inhibiting mtDNA replication and/or expression will force a cellular energy metabolism shift to anaerobic glycolysis. This change is exacerbated in the phenotype of mtDNA-depleted (rho0) cells that require exogenous pyruvate and uridine for their survival and proliferation (King MP, et al., Science. 1989;246(4929):500-3). Pyruvate is needed to replenish cellular NAD⁺ through lactate dehydrogenase activity (King et al, 1989), as well as to support aspartate biosynthesis (Birsoy K, et al., Cell. 2015;162(3):540-51) when the mitochondrial respiratory capacity is lacking or severely reduced. Uridine complements the lack of endogenous pyrimidine biosynthesis due to stalling of the dihydroorotate dehydrogenase (DHODH) activity which is coupled to the respiratory chain to carry out the efficient oxidation of mitochondrial ubiquinol (Loffler M, et al., Nucleosides, Nucleotides & Nucleic acids. 2020:1-25).

Moreover, in the context of the increasing evidence of the involvement of mitochondria in the proper function of the adaptive and innate immune system (Pearce EL, et al., Science. 2013;342(6155):1242454; West AP, et al., Nature Reviews Immunology. 2011;11(6):389-402), immune dysfunction and an increased incidence of infections have been reported in patients with mitochondrial diseases (Walker MA, et al., Journal of Immunology Research. 2014;2014:164309; Kapnick SM, et al., Metabolism: clinical and experimental. 2018;81:97-112).

In this context, clinical and experimental data have recently demonstrated that the immune system and, specifically, T cells require a functional mitochondrial respiratory chain (Tarasenko TN, et al. Cell metabolism. 2017;25(6):1254-68 e731). Firstly, a retrospective analysis showing recurrent or severe infections in a cohort of pediatric patients with mitochondrial diseases prompted the authors to characterize their T cell populations. The work revealed a baseline paucity of memory T cells as well as leukopenia provoked by episodes of acute infections. Then, they created a mouse model with a T cell-specific knockout of the *COX10* gene (*TCox10*^{*-*/*-*}) encoding an essential assembly factor for cytochrome c oxidase, i.e. the terminal enzyme of the mitochondrial respiratory chain. The *TCox10*^{*-*/*-*} mice displayed severe abnormalities in T cell activation, differentiation and function as well as an impaired immune response to vaccines and viral infections. In line with the above findings, since the three catalytic subunits of cytochrome c oxidase are encoded by mtDNA, mitotoxic antibiotics by lowering the mtDNA copy number (as in the case of fluoroquinolones), or by inhibiting the mitochondrial protein synthesis, would have a detrimental impact on T cells.

The above evidence clearly show a direct causal relationship between mitochondrial disease and immunodeficiency (see also Reichenbach J, Schubert R, Horvàth R, et al. Fatal neonatal-onset mitochondrial respiratory chain disease with T cell immunodeficiency. Pediatr Res. 2006;60(3):321-326). However, systemic mitochondrial dysfunctions have been associated to a plethora of pathological conditions, such as Down syndrome (Valenti D, Braidy N, De Rasmo D, et al. Mitochondria as pharmacological targets in Down syndrome. Free Radic Biol Med. 2018;114:69-83), atherosclerosis (Shemiakova T, Ivanova E, Grechko AV, Gerasimova EV, Sobenin IA, Orekhov AN. Mitochondrial Dysfunction and DNA Damage in the Context of Pathogenesis of Atherosclerosis. Biomedicines. 2020;8(6):166), as well as, diabetes and obesity (Galgani JE, Fernández-Verdejo R. Pathophysiological role of metabolic flexibility on metabolic health [published online ahead of print, 2020 Aug 19]. Obes Rev. 2020;10.1111/obr.13131).

Interestingly, different forms of immunodeficiencies have been also described to occur in Down's syndrome (Bloemers BL, van Bleek GM, Kimpen JL, Bont L. Distinct abnormalities in the innate immune system of children with Down syndrome. J Pediatr. 2010;156(5):804-809.e5.), obesity (Milner JJ, Beck MA. The impact of obesity on the immune response to infection. Proc Nutr Soc. 2012;71(2):298-306.) and diabetes (Galgani et al., cf. above), often leading to a higher risk of recurrent infections in the corresponding patients.

Finally, the decay of mitochondrial function and immunosenescence are co-existing and possibly related hallmarks of human aging (McGuire PJ. Mitochondrial Dysfunction and the Aging Immune System. Biology. 2019;8(2)).

US 2004/0224920 A1 and WO 00/50043 A1 are directed to methods for the treatment of mitochondrial disorders, particularly those associated with a pyrimidine deficiency, and most particularly those associated with a deficiency in uridine. The pharmaceutical agents include pyrimidine-based nucleosides such as triacetyluridine, or the like, wherein the authors also refer to the optional administration of co-factors such as calcium pyruvate. However, both references do not concern a boosting effect or protective effect on immune cells.

US 2002/0049182 A1 and WO 00/11952 A1 refer to a method for treating pathophysiological consequences of mitochondrial respiratory chain deficiency in a mammal, wherein it is based on the discovery that administration of pyrimidine nucleotide precursors is effective in treatment of a large variety of symptoms and disease states related to mitochondrial dysfunction. Advantageous compounds are short-chain fatty acid esters of uridine or cytidine, wherein pyruvic acid is also useful for treatment of cells with defective mitochondrial function. According to the authors, uridine tripyruvate (2',3',5'-tri-O-pyruvyluridine) provides the benefits of both pyrimidines and pyruvate. However, both references do not concern any boosting effect or protective effect on immune cells.

### Object

Since the inventors received evidence that the effect of impaired proper mitochondrial function is more marked in actively dividing cells, such as the immune system cells, the object of the present invention is to provide compositions for regulating the proliferation, activity and survival of immune cells in connection with immunodeficiencies caused by impaired cellular respiration.

### Brief Description of the Present Invention

This object is solved by the subject-matter of independent claim 1. Preferred embodiments are mentioned in the dependent claims.

The invention provides a composition comprising a therapeutically effective amount of both an uridine and pyruvate compound as claimed in claim 1 for use in a method of regulating the proliferation, activity and survival of immune cells to ameliorate and/or prevent immunodeficiencies caused by impaired cellular respiration. Examples of immunodeficencies caused by impaired cellular respiration are those caused by mitochondrial dysfunction, i.e. mitochondrial immunodeficencies. Furthermore, impairment of cellular respiration can also occur under hypoxic/anoxic conditions, shortening of reducing substrate supply as well as alteration of energy metabolism.

The present invention is based on the causal connection between mitochondrial respiratory dysfunctions and immunodeficiency. Mitochondrial dysfunction occurs during aging, mitochondrial diseases and/or other pathological conditions associated with mitochondrial dysfunctions. As a further example, anti-HIV/AIDS drugs, such as zidovudine (AZT), are mitotoxic, thus possibly worsening the already devastating immunodeficiency of HIV patients. The composition of the present invention is suitable to protect and/or boost the (residual) immunocompetence, even more if the patients undergo mitotoxic therapies. Furthermore, statins, widely used drugs that reduce serum cholesterol levels, have been clearly shown to inhibit mitochondrial respiratory chain, hence, mitochondrial function. This adverse effect of statins has been mostly studied on muscle since myopathies are the best characterized diseases caused by side-effects of these drugs.

Immune cells represent the favorite target of uridine and pyruvate when mitochondria are challenged due to their high proliferation rate (as compared to post-mitotic tissues). Thus, the present invention is useful for ameliorating or preventing (primary and/or secondary and/or acquired) mitochondrial immunodeficiencies through immune cell boosting.

Moreover, protecting the immune system in patients with mitochondrial diseases or during mitotoxic therapies could be of the utmost importance. In addition, the administration of uridine and pyruvate compounds has a positive effect in subjects, in particular elderly people over the age of 50, as a general improvement of immunosenescence. The administration may also ameliorate the response of the immune system to vaccines, in particular in elderly people over the age of 50.

The inventors have found out that many classes of medicaments interfere with mitochochondrial protein synthesis and/or function, causing a reduction of the energy generating system (enzymes of the oxidative phosphorylation system, OXPHOS). Fast dividing cells (e.g. immune cells) are mostly affected, as they cannot keep the number of OXPHOS complexes constant in the daughter cells. Thus, it is known from other studies that enzymes of OXPHOS are diluted as they cannot be re-synthesized under drug influence. Within 4-5 cell divisions, the number of OXPHOS complexes is under the level needed to sustain cells energy demand (approximately 8-12 %) so that a biochemical pathway shift to glycolysis will occur. In contrast, cells in resting state are less affected as they display turnover rates (half-life) of mitochondrially synthesized proteins of 8-20 days. Thus, they can cope with the blocked biosynthesis of OXPHOS so that even after a 30 days treatment, symptoms cannot be detected within this time span.

Hence, the impairment of mitochondrial function by aging, mitochondrial diseases and/or other pathological conditions associated with acquired mitochondrial dysfunctions (e.g. mitotoxic drugs) are caused by two distinct ways:
- Firstly, due to the inhibition of mitochondrial protein synthesis, these medicaments shift the energy generating pathway towards glycolysis. Thus, glycolysis needs to cover almost the entire energy demand of cells and needs to function at its best.
- Secondly, due to the diminished rate of OXPHOS complexes, a second problem, distinct from the energy underrun, kicks in: electrons from the dihydroorotate dehydrogenase (DHODH) are fed exclusively into the OXPHOS via the mitochondrial ubiquinol/ubiquinone cycle. If OXPHOS enzymes become rate limiting, the DHODH pathway comes to a halt as electrons cannot be released from the DHODH-redox reaction. The enzyme (DHODH) is linked to the essential uridine biosynthesis pathway. Thus, shortening in cell's uridine supply causes consequently a stalling of the cell cycle.

When metabolism is shifted towards anaerobic glycolysis, cells produce lactate from pyruvate that is released into the blood. As a consequence of the occurring metabolic switch, the NAD⁺/NADH ratio is shifted towards NADH, because it cannot be re-oxidized by the few remaining OXPHOS complexes. This situation leads to a shortening of NAD⁺ and pyruvate within the cell so that essential biochemical pathways are blocked. When pyruvate is administered, both problems are solved:
- Firstly, the inner cellular pyruvate pool increases so that stalled biochemical reactions can be restarted again
- Secondly, as the NADH level can be reduced by production of lactate from the administered pyruvate, reactions previously stalled by the high NADH level can be restarted again.

Cell's metabolism is additionally challenged by the diminished uridine pool that is caused by the stalled DHODH reaction. It is known that cells uridine pool is filled by three distinct pathways:
- Firstly, the cell is fed from the salvage pathway. That is: all uridines (pyrimidines) utilized within the cell (e.g. the building blocks all nucleic acids or UDP release from UDP-glucuronic acid) are reused upon breakdown. The pyrimidine level resulting from the recycling is sufficient to sustain cell's demand in a resting cell.
- Secondly, the pyrimidine hunger of dividing cells can be saturated from the freshly synthesized building blocks via the DHODH pathway.
- Thirdly, pyrimidines can be taken up by the cell. Thus extracellular sources can additionally bring in the required building blocks for cells in mitosis.

When uridine (pyrimidine) is administered, cell's uridine pool is refilled.

Thus, administration of both of pyruvate and uridine ameliorates or prevents immunodeficiencies caused by impaired cellular respiration.

### Brief Description of the Figures:

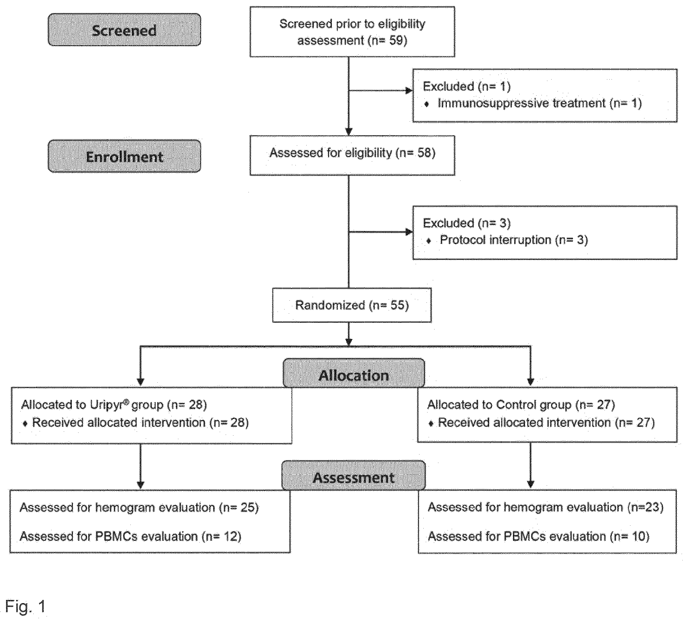

Figure 1: Flow chart for study population selection.

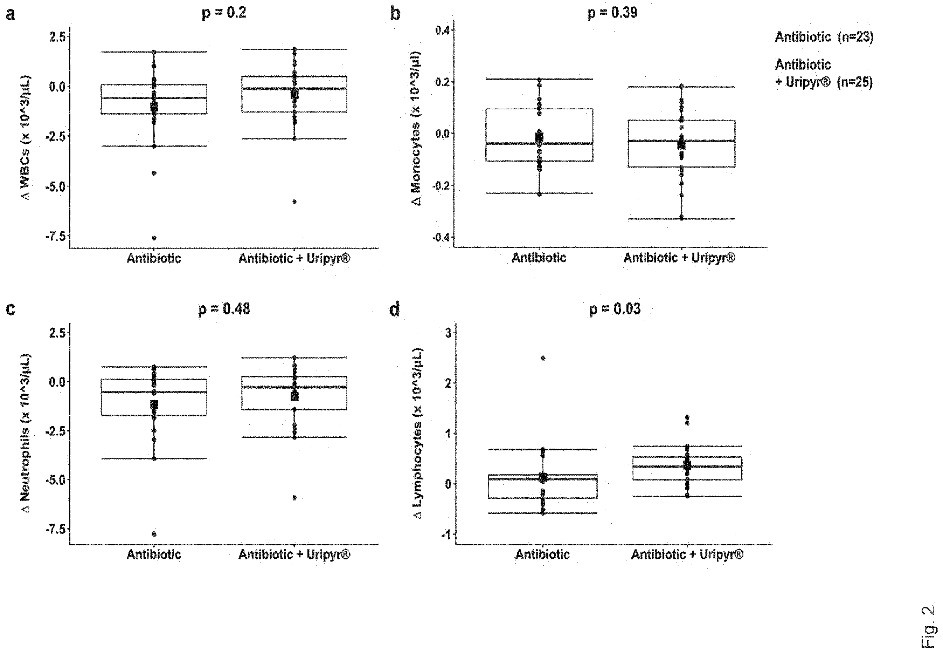

Figure 2: Modulation of blood count by Uripyr^{®} oral supplementation in mitotoxic antibiotic-treated patients. Differential value of White Blood Cells (WBCs, **a),** monocytes **(b),** neutrophils **(c)** and lymphocytes **(d)** counts, calculated as delta value between the end of the antibiotic treatment (T_{END}) and the baseline (T₀) in the Uripyr^{®} and the control group. Data are expressed as median and [IQR] for a and d and mean ± SD for b and c. *p<0.05

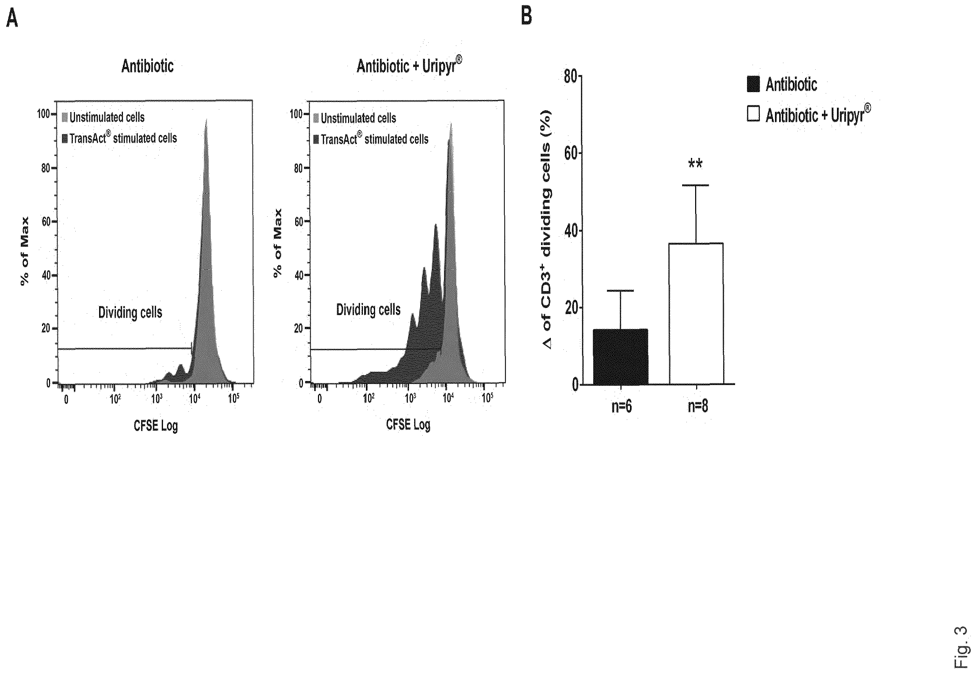

Figure 3: Effects of Uripyr^{®} oral supplementation on the ex *vivo* proliferation of CD3⁺ gated cells from PBMCs of mitotoxic antibiotic-treated patients. (a) Representative overlay histograms for CFSE staining of CD3⁺ cells gated from PBMCs of the Uripyr^{®} and control group patients. The histograms indicate the proliferating ability of unstimulated (grey histograms) and TransAct^{®} stimulated (black histograms) cells. (b) The mean percentage of dividing CD3⁺ gated cells from PBMCs was calculated as delta value of TransAct^{®} stimulated versus unstimulated cells after antibiotic treatment - with and without Uripyr^{®} supplementation (white and black bars, respectively). Data are expressed as mean ± SEM. ***p≤0.01

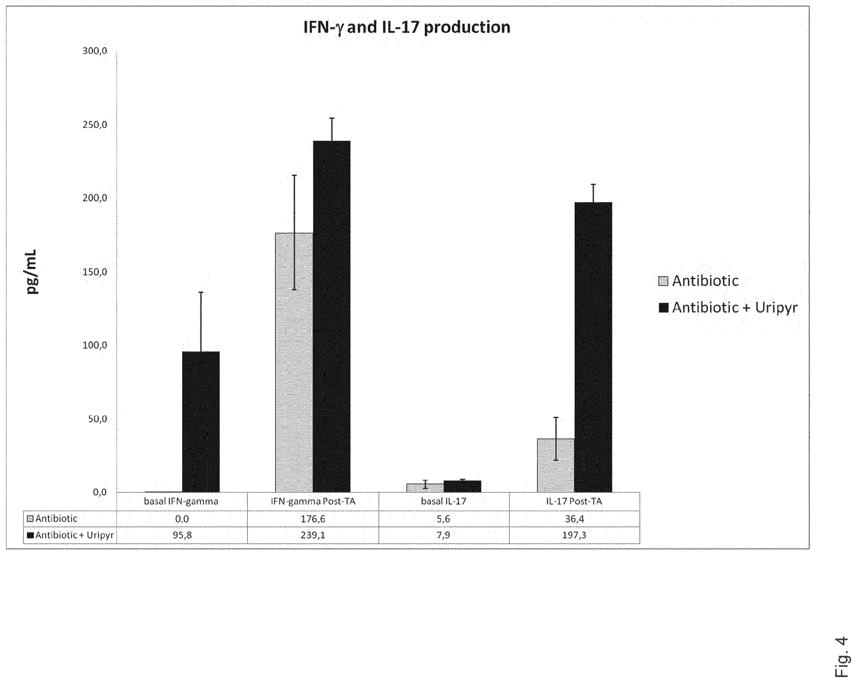

Figure 4: Effect of Uripyr^{®} oral supplementation on the production of IFN-gamma and IL-17 production by *ex vivo* cultured PBMCs in mitotoxic antibiotic-treated patients.

### Detailed Description of the Present Invention

The invention provides a composition comprising a therapeutically effective amount of both an uridine and pyruvate compound as claimed in claim 1 for use in a method of regulating the proliferation, activity and survival of immune cells to ameliorate and/or prevent immunodeficiencies that are caused by impaired cellular respiration. Examples of immunodeficencies caused by impaired cellular respiration are those based on mitochondrial dysfunction, i.e. mitochondrial immunodeficencies. The regulating of the proliferation, activity and survival of immune cells is preferably achieved by increasing the production of cytokines.

In the present invention the terms "formulation" and "composition" are used interchangeably. In a preferred embodiment it is a dietary supplement or nutritional supplement. In a particular preferred embodiment it is "URIPYR"^{®} (Mitobiotix, Italy). URIPYR^{®} is a composition comprising calcium pyruvate and uridine-5'-monophosphate-disodium together with pharmaceutical acceptable carriers.

"Dietary or nutritional supplement" as used herein refers to a product intended to supplement or complement a subject's diet, the product comprising one or more substances with a nutritional and/or physiological effect on a subject. The dietary supplement can be a partial nutritional composition, which does not contain all the essential macro- and micronutrients and hence may not be used to replace one or more daily meals and/or may not be used as the sole source of nutrition of a subject. The dietary supplement may be a liquid, powder, gel, paste, solid, tablet, capsule, concentrate, suspension or ready-to-use formulation.

"Subject" as used herein refers to humans and non-human primates (e.g. guerilla, macaque, marmoset), livestock animals (e.g. sheep, cow, horse, donkey, pig), companion animals (e.g. dog, cat), laboratory test animals (e.g. mouse, rabbit, rat, guinea pig, hamster), and any other organism who can be benefit from the agents of the present disclosure. There is no limitation on the type of animal that could benefit from the presently described agents. A subject regardless of whether it is a human or non-human organism may be referred to also as a patient, individual, animal, host, or recipient.

The term "about" as used herein, means in quantitative terms plus or minus 5%, or in another embodiment plus or minus 10%, or in another embodiment plus or minus 15%, or in another embodiment plus or minus 20%.

It has been found out by the inventors that it is possible to ameliorate and/or prevent immunodeficiencies that are caused by impaired cellular respiration by the supplementation with an uridine compound and pyruvate compound as defined above. The term "an uridine and pyruvate compound" also encompasses two or more of each of them or mixtures or two or more of each.

The term "immunodeficiency caused by impaired cellular respiration" means defective survival, activity and/or replication of immune cells caused by a defective cellular oxygen consumption. Examples of immunodeficencies caused by impaired cellular respiration are those based on mitochondrial dysfunction, i.e. mitochondrial immunodeficencies. These mitochondrial immunodeficencies may be primary, secondary or induced/acquired mitochondrial immunodeficencies.

"Primary mitochondrial immunodeficiencies" are those immunodeficiencies detected in patients with pathologies caused by mitochondrial dysfunctions. Examples of primary mitochondrial immunodeficiencies are genetic diseases caused by mutations in mitochondrial DNA (MERF, MELAS, LHON, etc) or by mutations in nuclear genes encoding proteins with mitochondrial localization/function (mitochondrial DNA depletion syndromes, CPEO, Friedreich Ataxia, neuromuscular disorders, cardiomyopathies, etc.).

"Secondary mitochondrial immunodeficiencies" are those immunodeficiencies detected, or still unexplored, in pathological conditions associated (but not causally related) to mitochondrial dysfunctions. Examples of secondary mitochondrial immunodeficiencies can be those found in Down's syndrome, diabetes, obesity and aging.

"Induced/acquired mitochondrial immunodeficiencies are caused by drugs, toxins or any substance causing mitochondrial dysfunction (mitotoxicity), including hypoxic/anoxic conditions, e.g. those associated with defective blood circulation and/or oxygen delivery.

"Immune cells" mean all the cells belonging to the immune system, particularly lymphocytes, more particularly T-cells and B-cells. The T-cells (CD3 lymphocytes) are part of the cellular immune system and represent a subgroup of the lymphocyctes which are in turn a subgroup of the white blood cells. About 70% of the lymphocytes in the blood are T-cells. The characteristic feature of these cells is that they carry the CD3 antigen at their surface. The T- cells are involved in important roles in the following disease processes: (i) Defense against viruses, fungi and certain bacteria (e.g. tuberculosis); (ii) defense against tumor cells; (iii) certain allergic reactions of the delayed type; (iv) graft-versus-host reactions.

The T-cells as such may be divided into some subgroups:
- T-helper cells (CD3 and CD4 positive),
- T-suppressor cells (CD3 and/or CD8 positive),
- Cytotoxic T-Cells (CD8 positive)
- Regulatory T-Cells
- T memory cells
- "NK-like" T-cells (CD3, CD16 and CD56 positive),
- Aktivated T-cells (CD3 und HLA-DR positive)

In particular T cells, upon antigen recognition, undergo rapid clonal expansion and differentiate into specific effector subsets whose functions are counteracted by regulatory T cells (Treg). After these processes, only long-living memory T cells survive and can promote a faster and stronger response to a secondary challenge of the same nature. Therefore, mitochondria may be seen as master metabolic regulators of T lymphocytes thanks to their ability to modulate different stages of T cell adaptive responses including migration, activation, proliferation, differentiation, memory phase, and exhaustion. Naïve T cells predominantly use OXPHOS as the principal source of energy, while activated T cells exhibit higher glycolysis. During the T cells differentiation process, a shift towards aerobic glycolysis, induces the generation of proinflammatory T cell subsets (Th1 and Th17), while the promotion of OXPHOS leads to the onset of a regulatory phenotype for T cells (Treg and memory T cells).

The B cells, also known as B lymphocytes, are a type of white blood cell of the lymphocyte subtype. They function in the humoral immunity component of the adaptive immune system by secreting antibodies. Additionally, B cells present antigens (they are also classified as professional antigen-presenting cells (APCs)) and secrete cytokines. In mammals, B cells mature in the bone marrow. Types of B-cells are (i) Plasma cells, (ii) Lymphoplasmacytoid cell, (iii) Memory B cell; (iv) B-2 cells, Folicular B cells and Marginal Zone B cells; (v) B-1 cells and (vi) Regulatory B (Breg) cells.

As used herein, the term "cytokine" relates to a category of signalling molecules that are used extensively in cellular communication. They comprise proteins, peptides, or glycoproteins. The term "cytokine" encompasses a large family of polypeptide regulators that are produced widely throughout the body by cells of diverse embryological origin. The action of cytokines may be autocrine, paracrine, and endocrine. All cytokines are critical to the development and functioning of both the innate and adaptive immune response. They are often secreted by immune cells that have encountered a pathogen, thereby activating and recruiting further immune cells to increase the immune system's response to the pathogen. Preferably, the cytokine that is induced through the administration of uridine and pyruvate in the present invention is an interferon or interleukin. All interferons (IFNs) are natural cell-signalling proteins produced by the cells of the immune system of most vertebrates in response to challenges such as viruses, parasites and tumor cells. Interferons assist the immune response by inhibiting pathogen replication within host cells, activating natural killer cells and macrophages, increasing antigen presentation to lymphocytes, and inducing the resistance of host cells to viral infection. All interferons in general have several effects in common and, accordingly, the result of increasing the production of IFN-gamma by administering uridine and pyrivate to a patient might apply to further interferons. Interferons are antiviral and possess antioncogenic properties, macrophage and natural killer cell activation, and enhancement of major histocompatibility complex glycoprotein classes I and II, and thus presentation of foreign (microbial) peptides to T cells. The metabolism and excretion of interferons take place mainly in the liver and kidneys. They rarely pass the placenta but they can cross the blood-brain barrier.

There are three major classes of interferons that have been described for humans:
(a) Interferon type I: The type I interferons present in humans are IFN-alpha, IFN-beta and IFN-omega.
(b) Interferon type II: In humans this is IFN-gamma.
(c) Interferon type III: Signal through a receptor complex consisting of IL10R2 (also called CRF2-4) and IFNLR1 (also called CRF2-12)
Interleukins (ILs) are a group of cytokines (secreted proteins and signal molecules) that were first seen to be expressed by white blood cells (leukocytes). ILs can be divided into four major groups based on distinguishing structural features. However, their amino acid sequence similarity is rather weak (typically 15-25% identity). The human genome encodes more than 50 interleukins and related proteins. The function of the immune system depends in a large part on interleukins, and rare deficiencies of a number of them have been described, all featuring autoimmune diseases or immune deficiency. The majority of interleukins are synthesized by helper CD4 T lymphocytes, as well as through monocytes, macrophages, and endothelial cells. They promote the development and differentiation of T and B lymphocytes, and hematopoietic cells. As could be shown in the present invention the administration of pyruvate and urdine premotes the production of interleukin 17 (IL-17). It is a potent cytokine produced by activated memory T cells. The IL-17 family is thought to represent a distinct signalling system that appears to have been highly conserved across vertebrate evolution.

"Mitotoxic substances" are substances causing mitochondrial dysfunctions.

"Mitochondrial disease" are disease caused by mitochondrial dysfunctions.

"Pathological conditions associated with mitochondrial dysfunctions" are diseases associated to, but not primarily caused by, mitochondrial dysfunctions (e.g. obesity, diabetes, Down's syndrome, ageing).

"Mitochondrial dysfunction" is characterized by a diminished or even destroyed generation of ATP through OXPHOS. This may be caused by inhibiting mtDNA replication and/or expression that will force a cellular energy metabolism shift to anaerobic glycolysis. This change is exacerbated in the phenotype of mtDNA-depleted (rho0) cells as an extreme.

The dosage of the composition according to the invention is determined by patient-specific parameters, such as age, weight, sex, severity of the disease, *etc.* The mode of treatment and the dosage regimen of the uridine and pyruvate compounds will be mainly dictated by the specific symptoms. The effective dosage will vary somewhat from patient to patient, and will depend upon the condition of the patient and the dosage form.

The terms "dosage regimen" or "mode of treatment" refer to a timely sequential or simultaneous administration of the components of the composition of the present invention. This means that the components may be provided in a unit dosage form with physical contact to each other (e.g. one single tablet or solution) or as separate entities (e.g. two tablets or solutions) to be taken simultaneously or with a certain time difference. This time difference may be between 0.5 hour and 1 day, preferably between 1 hour and 5 hours. In one embodiment, the uridine and/or pyruvate are physically segregated within the dosage form, for example, separated by an acrylic or cellulose membrane in a tablet or capsule. In an alternative embodiment, both ingredients are in physical contact within the dosage form, such as for example, in a mixed powder or granules form (free or formed into a tablet or capsule) or a liquid. The mode of administration is preferably simultaneously, i.e. at substantially the same time.

In the present invention the uridine compound is administered to increase a level of uridine in a tissue, plasma, or cell of a subject.

Generally, the term "uridine" or "uridine compound" means uridine, uridine phosphate(s) or uracil. Examples are poly-U, any C/U-containing oligonucleotide and RNA/DNA, respectively.

According to the present invention the uridine compound that is administered is disodium salt of uridine-5'monophosphate (UMP).

Other suitable compounds which, however, are not encompassed by the present claims, are a uridine-5'-diphosphate (UDP), a uridine-5'triphosphate (UTP) or UDP-glucose. Further suitable compounds which, however, are not encompassed by the present claims, are a cytidine-5'monophosphate, a cytidine-5'-diphosphate (CDP), a CDP-glucose, cytidine-diphosphocholine (CDP-choline; citicholine), acylated uridine, acyl derivatives of uridine or mixtures thereof (e.g. those disclosed in U.S. Pat. No. 5,470,838), orotic acid or orotidylate.

In addition to the disodium salt of uridine-5'monophosphate (UMP), a mixture of two or more of the above mentioned uridine compounds may be administered.

In one embodiment, the uridine compound is administered in a dosage of between about 20 milligrams (mg) and 50 grams (g) per day. In another embodiment, the uridine compound is administered in a dosage of about 50 mg-30 g per day. In another embodiment, the dosage is about 75 mg-20 g; 100 mg-15 g; 100 mg-10 g; 200 mg-8 g; 400 mg-6 g; 600 mg-4 g; 800 mg-3 g; 1-2.5 g; 1.5-2 g, about 2 g per day.

According to the present disclosure the "pyruvate" or "pyruvate compound" is pyruvic acid, a salt thereof (e.g. methyl or ethyl pyruvate) or creatine pyruvate. Additionally, all compounds that can be easily converted within the cell to pyruvate, e. g. phosphoenolpyruvate, 2-phosphoglycerate, 3-phosphoglycerate, 1,3-biphosphoglycerate, glyceraldehyde 3-phosphate, dihydroxyacetone phosphate, alanine, serine, cystein, glycine, tryptophan, asparagine, aspartate, methionine, malate or oxalacetate are disclosed but not encompassed by the present claims. According to the present invention the pyruvate compound is calcium salt of pyruvate.

In one embodiment, the pyruvate compound is administered in a dosage of between about 100 milligrams (mg) and 10 grams (g) per day. In another embodiment, the pyruvate compound is administered in a dosage of about 200 mg-9 g per day. In another embodiment, the dosage is about 500 mg-8 g; 750 mg-7 g; 1-6.5g, 1.5-6 g, 2-5 g, or 2.5-3.5 g per day.

The pharmaceutical composition of the present invention may be prepared and administered in any dosage form suitable for administration to the subject's body. It is formulated to be compatible with its intended route of administration. Examples of routes of administration include parenteral (e.g. intravenous, intradermal, subcutaneous), oral and systemic (e.g. topical, transmucosal) administration. According to the present invention the oral administration is preferred.

In a preferred embodiment, the formulation may be prepared as a tablet, powder, capsule, liquid, gel, a suspension, an emulsion or a solution.

Solutions or suspensions used for parenteral, intradermal, or subcutaneous application can include the following components: a sterile diluents such as water for injection, saline solution, fixed oils, polyethylene glycols, glycerine, propylene glycol, or other synthetic solvents; antibacterial agents such as benzyl alcohol or methyl parabens; antioxidants such as ascorbic acid or sodium bisulfate; chelating agents such as ethylenediaminetetraacetic acid; buffers such as acetates, citrates, or phosphates and agents for the adjustment of tonicity such as sodium chloride or dextrose. The pH can be adjusted with acids or bases, such as hydrochloric acid or sodium hydroxide. The parenteral preparation can be enclosed in ampoules, disposable syringes, or multiple dose vials made of glass or plastic.

Pharmaceutical or nutritional compositions suitable for an injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor^{®} EL (BASF, Parsippany, N.J.), or phosphase buffered saline (PBS). In all cases, the composition must be sterile and should be fluid to the extent that easy syringability exists. It should be stable under the conditions of manufacture and storage and must be preserved against the contaminating action of microorganism such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. Prevention of the action of microorganism can be achieved by various antibacterial and antifungal agents, for example, parabens, chlorobutanol, phenol, ascorbic acid, thimerosal, and the like. According to embodiments, isotonic agents, for example, sugars, polyalcohols such as manitol, sorbitol, or sodium chloride in the composition are added. Prolonged absorption of the injectable compositions can be brought about by including in the composition an agent which delays absorption, for example, aluminum monostearate and gelatin.

Sterile injectable solutions can be prepared by incorporating the active compound in the required amount in an appropriate solvent with one or a combination of ingredients enumerated above, as required, followed by filtered sterilization. Generally, dispersions are prepared by incorporating the active compound into a sterile vehicle which contains a basic dispersion medium and the required other ingredients from those enumerated above. In the case of sterile powders for the preparation of sterile injectable solutions, the preparation is prepared by vacuum drying or freeze-drying, which yields a powder of the active ingredient plus any additional desired ingredient from a previously sterile-filtered solution thereof.

In a further preferred embodiment the formulation of the present invention is suitable for oral administration. Oral compositions generally include an inert diluents or an edible carrier. For the purpose of oral therapeutic administration, the active compound can be incorporated with excipients and used in the form of powder, tablets, troches, chewie, gel caps, soft gel or capsules, e.g. gelatin capsules. Oral compositions can also be prepared using a fluid carrier for use as a mouthwash. Pharmaceutically compatible binding agents, or adjuvant materials can be included as part of the composition. The tablets, pills, capsules, sachets, troches and the like can contain any of the following ingredients, or compounds of a similar nature: a binder such as microcrystalline cellulose, gum tragacanth or gelatin; an excipient such as starch or lactose, a disintegrating agent such as alginic acid. Primogel, or corn starch; a lubricant such as magnesium stearate or sterotes; a glidant such as colloidal silicon dioxide; a sweetening agent such as sucrose, maltodextrin or saccharin; antioxidants such as citric acid or ascorbic acid, or a flavoring agent such as natural aromas, peppermint, vanilla, strawberry, cherry, grape, lemon, or orange flavoring.

Systemic administration can also be transmucosal or transdermal. For transmucosal or transdermal administration, penetrants appropriate to the barrier to be permeated are used in the formulation. Such penetrants are generally known in the art, and include, for example, for transmucosal administration, detergents, bile salts, and fusidic acid derivatives. Transmucosal administration can be accomplished through the use of nasal sprays or suppositories. For transdermal administration, the active compounds are formulated into ointments, salves, gels, or creams as generally known in the art. The compounds can also be prepared in the form of suppositories (e.g., with conventional suppository bases such as cocoa butter and other glycerides) or retention enemas for rectal delivery.

The formulation of the present invention may include any additional component as desired, as long as such components do not substantially erode the effectiveness of the uridine and pyruvate compounds. Such components may include, for example, thickeners, sweeteners, flavorants, fragrances, additional pharmaceuticals, glycine, mannitol, silicone dioxide, silica gels, binders, vitamins, carriers (such as, for example, glycerin, starches, celluloses, chitins, starches, water, alcohol) and minerals.

In the example part of the present application the inventors focused their attention on the effects of mitotoxic antibiotics on immune cells where the antibiotics have been chosen just as exemplary model initiators of pathological conditions associated with impaired cellular respiration, i.e. conditions comparable to those of acquired mitochondrial dysfunctions. However, the described effects may be extrapolated to any mitochondrial disease or other pathological conditions associated with acquired or inherited mitochondrial dysfunctions or acquired or inherited mitochondrial immunodeficiencies. Due to their off-target inhibition of mitochondrial protein synthesis and DNA replication and transcription, as well as, of mitochondrial respiratory chain function, mitotoxic drugs can cause actively dividing cells to become dependent on uridine and pyruvate supplementation for their growth, as in the case of mtDNA-depleted (rho0) cells. In this context, the inventors carried out a clinical pilot study in 55 patients with asymptomatic bacteriuria (ABU) and positive sperm cultures undergoing treatment with mitotoxic antibiotics, with or without uridine+pyruvate (Uripyr^{®}) supplementation. The data, obtained both *in vivo* and by a modified cell proliferation assay of isolated PBMCs cultured *ex vivo* in autologous serum, show a striking protection of the T cell proliferative capacity in the Uripyr^{®} supplementation group.

Besides its classical role in RNA and DNA synthesis, uridine can have multi-targeted effects due to its conversion or incorporation in other molecules with different biological actions. Therefore, the inhibition of endogenous uridine biosynthesis by mitotoxic substance can be associated with other tissue-specific adverse effects. For instance, uridine triphosphate (UTP) is used to activate glucose-1P to UDP-glucose, then oxidized to UDP-glucuronate, required for the biosynthesis of glycosaminoglycans, such as heparan sulfate, chondroitin sulfate and hyaluronic acid. These essential components of the extracellular matrix interact with collagens and other proteins, thus playing an essential role in connective tissue function.

It is shown in the present invention that supplementation with uridine and pyruvate protects the proliferative capacity of T lymphocytes, effector cells of cell-mediated immunity, from mitotoxic therapies. The inventors showed in a cytofluorimetric analysis of CD3⁺ cells gated from total PBMCs a 2.6-fold increase in the number of dividing cells in the uridine/pyruvate treated group as compared to the control group. This result demonstrates that the uridine/pyruvate supplementation sustains and protects the T cell proliferative capacity. In addition, the inventors showed in 48 patients a 3.4-fold significant difference in the lymphocytes differential count in the uridine/pyruvate treated group versus the control group.

Moreover, protecting the immune system in patients with mitochondrial diseases or during mitotoxic therapies could be of the utmost importance. In addition, the administration of uridine and pyruvate compounds has a positive effect in subjects, in particular elderly people, as a general improvement of immunosenescence. Thus, the administration may ameliorate the response of the immune system to vaccines, in particular in elderly people over the age of 50. In this regard it may be advantageous to add uridine and pyruvate compounds as adjuvants to vaccines or to administer them with a close time difference (e.g. within the same day) prior to or after the vaccine.

In addition, it has been shown in the present invention that the supplementation with uridine and pyruvate compounds increases the production of cytokines (e.g. IFN-γ and/or IL-17) in patients. These cytokines boost and protect T cell function which is important for a normal functioning of the patient's immune system. Distracting mitochondrial translation in differentiating T cells compromises the integrity of the respiratory chain. This may result in deficient oxidative phosphorylation, dimininishing NAD⁺ concentrations and impairing cytokine production in differentiating T cells. As shown in the present invention, however, in humans these effects can be reversed by the administration of uridine and pyruvate compounds.

The following examples show preferred embodiments of the present invention.

### Example 1: Protection of the proliferative capacity of immune T cells by uridine + pyrivate

### Study participants

A population of subjects scheduled to undergo endourological maneuvers, prostate biopsy or medically-assisted reproduction was selected in the Urology, Andrology, and Kidney Transplantation Units of the University of Bari. Among them, patients with asymptomatic bacteriuria or with positive sperm culture requiring antibiotic treatment were enrolled according to EAU guidelines. The population did not include patients with acute and/or life-threatening diseases like renal and hepatic failure, nor patients with infections, acute stress, or those in treatment with medications affecting the number of leukocytes (i.e. corticosteroids). Other exclusion criteria were: infections sustained by Multi-Drug Resistant (MDR), Xtreme Drug-Resistant (XDR), Pan Drug-Resistant (PDR) microbes; chronic systemic inflammatory diseases; neoplastic diseases of recent onset (less than 10 years) and/or under chemotherapeutic treatment; immune-depression conditions; pregnant and/or puerperal women; allergy and/or adverse reactions to the prescribed drug or its excipient. The study was conducted in compliance with the Helsinki Declaration and ethical approval was obtained from the Local Ethics Committee (protocol n° 5251). Between July 2017 and December 2019, 59-sequential eligible patients were recruited and after obtaining informed consent, 55 were finally enrolled in the trial. All the selected subjects belonged to Caucasian ethnicity and were predominantly men (85.18% of controls and 75% of the Uripyr^{®} group). The mean age of patients of the Urypyr group was 58.32±16.12 years vs. the 53.78±16.73 of controls, meanwhile the BMI was similar in the two groups (25.46±2.86 Uripyr^{®} group vs. 25.81±3.30 of controls). The percentages of chronic comorbidities and nephrological diseases did not differ considerably in the two experimental groups. The endpoint of the present study was the analysis of the effects of oral supplementation with Uripyr^{®} during treatment with mitotoxic antibiotics (= model agents for provoking mitochondrial deficiencies) on immune cell functional properties.

The enrolled patients underwent a complete blood count, urinalysis and urine and/or semen culture (according to the clinical judgment) at baseline (T₀), before starting any antibiotic treatment. Patients demographic and clinical data were also collected. Participants were randomly allocated to the "Antibiotic treatment plus Uripyr^{®}" (Uripyr^{®}) group or "Antibiotic treatment without Uripyr^{®}" (control) group. The randomization process and subsequent assignment to the intervention group was managed by an independent pharmacological consultant. Figure 1 shows a flow diagram of the population selection process. Patients in the Uripyr^{®} group were instructed to take three doses of Urypir^{®} (each containing 150 mg of uridine and 2 g of pyruvate) per day, one hour before taking the antibiotic. Each subject was treated with a single antibiotic according to the culture test susceptibility results and the duration and dosage described by the best-accepted guidelines (17-19) The mitotoxic antibiotics were administered at the following doses: levofloxacin 500 mg qd (7-10 days), ciprofloxacin 500 mg bid (7-10 days), tigecycline 50 mg bid, doxycycline 100 mg bid for the first day and then qd (7-10 days), linezolid 600 mg bid(10 days), erythromycin 1 g bid (10 days). Finally, at the end of the antibiotic therapy, each patient repeated the same clinical evaluations as at baseline (defined as T_{END} values).

### Proliferation test of CD3⁺ cells.

Peripheral blood mononuclear cells (PBMCs) were freshly isolated from venous blood drawn into BD Vacutainer^{®} Heparin Tubes using Ficoll Paque Plus (GE Healthcare, USA) according to the manufacturer's instructions. PBMCs were collected after the antibiotic treatment (T_{END}) with or without the Uripyr^{®} supplementation (Mitobiotix, Italy). PBMCs isolated from each patient were washed in sterile PBS 1X (Euroclone) and stained with CellTrace^{®} CFSE (Thermo Fisher Scientific, MA, USA) for 5 min at 37°C (1 µL/ml PBS1X). CFSE staining was stopped by diluting cells with five volumes of BSA1%-PBS1X (Bovine Serum Albumin, Sigma-Aldrich, USA) for 5 min at room temperature. Stained PBMCs from each patient were washed again in PBS1X and plated in 100% of autologous heat-inactivated serum for 30 min at 37°C, adjusting cell density to 1x10⁶ cells/ml in 48 wells. After seeding, cells were stimulated with 10µL of TransAct^{®} (Miltenyi Biotec, Bergisch Gladbach, Germany) according to the manufacturer's instructions and maintained at 37°C in a humidified atmosphere of CO₂/air (5:95). After 72hours of culture, cells were collected, washed in PBS1X and stained for 20 min with 1:20 diluted anti-CD3 antibody (CD3-PE-Vio 770, human, REA613 Clone - Miltenyi Biotec, Bergisch Gladbach, Germany). Cytofluorimetric analysis was carried out using the FC500 Flow cytometer (Beckman Coulter, CA, USA). Data analysis was performed using FlowJo^{®} software, version 10.6.1 (Becton, Dickinson and Company, OR, USA). Gating strategy: forward scatter vs. side scatter for PBMCs gating. Gated PBMCs were checked for positivity to CD3 (forward scatter vs. CD3). CD3⁺ cells were analyzed for CFSE staining by histogram to test their proliferating ability. The percentage of dividing cells from each population was calculated as a delta of the mean percentages of TransAct^{®} stimulated versus unstimulated cells (antibiotic treatment +/- Uripyr^{®}).

### Statistical methods

The clinical study was conducted with a 1:1 randomization according to the extension of the CONSORT Statement to randomized plot and feasibility trials (23). Statistical analyses were performed using the R statistical environment, packages "stats", "car", "gmodels", "fBasics" and "pwr". We performed a sample size calculation with a medium (0.5) and large (0.8) effect size, 80% power and two-sided 5% significance, yielding trial sample sizes of 64 and 26 subjects for each treatment arm, respectively. These evaluations may be useful for a future randomized control trial. Shapiro-Wilk tests and graphic evaluations of each variable were performed to test for normal distribution. All the quantitative variables were considered as normally distributed, except for differential values of white blood cells and lymphocytes. Descriptive statistics are presented as mean ± standard deviation and/or median and [IQR], for normally distributed and non-normally distributed continuous variables, respectively, whereas categorical variables are indicated as frequency (%). Then, Student t-test and Mann-Whitney's U test, as appropriate, were performed to assess comparisons among two groups. Differences in categorical variables between groups were assessed by Pearson χ² test. Plots and graphs were drawn up using the R package "graph" and "ggplot2" for the *in vivo* study and the GraphPad Prism statistical software release 6.1 for the ex *vivo* study. For ex *vivo* analysis, unpaired student t-test was used; data are expressed as mean ± SEM. A level of significance of p<0.05 was used to compare groups.

### Results

To understand the modulation by Uripyr^{®} oral supplementation on immune cells, the inventors performed an *ex vivo* proliferation test on cultured PBMCs from 14 patients. Lymphocyte proliferation assays are largely used in clinical diagnosis for monitoring the immune function efficiency (Froebel KS, et al., Journal of Immunological Methods. 1999;227(1-2):85-97.24). In this assay, *ex vivo* cultured lymphocytes are challenged with cell-specific activation and expansion *stimulus.* Specifically, the present ex *vivo* study was performed in a new experimental setting in which PBMCs isolated from Uripyr^{®} and control group patients at the end of the antibiotic treatment (T_{END}) were cultured in autologous serum, thus avoiding the exogenous supplementation of essential microelements and metabolites normally done with laboratory culture media as well as maintaining blood cells in their specific physiological environment. Since the proliferative *stimulus* utilized in the assay is specific for T cells, the inventors analyzed the cytofluorimetric profile of CD3⁺ cell populations in 8 patients from the Uripyr^{®} group and 6 patients from the control group. Cytofluorimetric analysis of CD3⁺ cells gated from total PBMCs showed a 2.6-fold increase in the number of dividing cells in the Uripyr^{®} group (36.56±5.29) as compared to the control group (14.23±4.15) (Figure 3, p≤0.01). This result demonstrates that Uripyr^{®} supplementation sustains and protects the T cell proliferative capacity. In addition, the differential blood cell count in 48 patients between the end of the antibiotic treatment (T_{END}) and the baseline (T₀) was calculated. The data show no significant difference in the White Blood Cells (WBCs) count when comparing the Uripyr^{®} group with the control group (Figure 2A). A similar trend was found for the monocytes and neutrophils counts (Figure 2B and C, respectively). By contrast, a 3.4-fold significant difference was found in the lymphocytes count showing a delta value of 0.34 [0.45] in the Uripyr^{®} group versus 0.10 [0.52] in the control group (Figure 2D, p<0.05).

### Example 2: Measurement of IFN-γ and IL-17 production

The study participants and the experimental setup for PBMCs isolation and ex-vivo culture in autologous serum was as in Example 1.

After 96 h of ex-vivo culture, without (basal) or with (Post-TA) the addition of TransAct, PBMCs isolated from a group of 8 patients at the end (T_{END}) of the antibiotic treatments with (n=4) and without (n=4) Uripyr^{®} supplementation, were collected for cytofluorimetric analysis and the supernatant autologous sera were immediately transferred to a clean polypropylene tube using a Pasteur pipette. Samples were stored at 80°C until analysis. for further analysis. Serum interferon-gamma (IFN-gamma) and IL-17A levels were measured by a sandwich enzyme-linked immunosorbent assay (ELISA) using the Human IFN gamma Platinum Kit Elisa (Invitrogen, Thermo Fisher Scientific, Inc.) and the Human IL-17A Platinum Kit Elisa (Invitrogen, Thermo Fisher Scientific, Inc.), respectively, according to the manufacturer's instructions.

As may be seen from Fig. 4, the supplementation with Uripyr^{®} leads to a substantial increase of the production of IFN-γ and IL-17 under basal and TransAct-stimulated conditions, respectively. These cytokines boost the T-cell function that had been impaired by the antibiotic administration. As mentioned above, the inventors focused their attention on the effects of mitotoxic antibiotics on immune cells where the antibiotics have been chosen just as exemplary model initiators of pathological conditions associated with impaired cellular respiration.

## Claims

1. A composition comprising a therapeutically effective amount of both an uridine compound and pyruvate compound together with a pharmaceutically acceptable ingredient for use in a method of regulating the proliferation, activity and survival of immune cells to ameliorate and/or prevent immunodeficiencies caused by impaired cellular respiration,
wherein the pyruvate compound is calcium salt of pyruvate, and the uridine compound is disodium salt of uridine-5'-monophosphate.

2. The composition for the use of claim 1, wherein the uridine and pyruvate compounds are in a unit dosage form to be administered simultaneously.

3. The composition for the use of claim 1 or 2, wherein it is provided as a dietary or nutritional supplement.

4. The composition for the use of any of claims 1 to 3, wherein the immune cells are T-cells or B-cells.

5. The composition for the use of any of claims 1 to 4, wherein the immunodeficiency caused by impaired cellular respiration is a mitochondrial immunodeficiency selected from a primary, secondary, or acquired mitochondrial immunodeficiency.

6. The composition for the use of claim 5, wherein the mitochondrial immunodeficiency is caused by immunosenescence in elderly people.

7. The composition for the use of any of claims 1 to 6, wherein the regulating of the proliferation, activity and survival of immune cells is achieved by increasing the production of cytokines.

8. The composition for the use of claim 7, wherein the cytokine is IFN-γ and/or IL-17.

## Patentansprüche

1. Zusammensetzung umfassend eine therapeutisch wirksame Menge sowohl einer Uridinverbindung als auch einer Pyruvatverbindung zusammen mit einem pharmazeutisch verträglichen Inhaltsstoff zur Verwendung in einem Verfahren zur Regulierung der Proliferation, der Aktivität und des Überlebens von Immunzellen, um Immuninsuffizienzen, die durch gestörte Zellatmung hervorgerufen werden, zu verbessern und/oder zu verhindern,
wobei die Pyruvatverbindung ein Calciumsalz von Pyruvat ist und die Uridinverbindung ein Dinatriumsalz von Uridin-5'-monophosphat ist.

2. Zusammensetzung zur Verwendung nach Anspruch 1, wobei die Uridin- und Pyruvatverbindungen in einer Einheitsdosierungsform vorliegen, um sie gleichzeitig zu verabreichen.

3. Zusammensetzung zur Verwendung nach Anspruch 1 oder 2, wobei sie als ein Diät- oder Nahrungsergänzungsmittel bereitgestellt wird.

4. Zusammensetzung zur Verwendung nach einem der Ansprüche 1 bis 3, wobei die Immunzellen T-Zellen oder B-Zellen sind.

5. Zusammensetzung zur Verwendung nach einem der Ansprüche 1 bis 4, wobei die Immuninsuffizienz, die durch gestörte Zellatmung hervorgerufen wird, eine mitochondriale Immuninsuffizienz, die aus einer primären, sekundären oder erworbenen mitochondrialen Immuninsuffizienz ausgewählt ist, ist.

6. Zusammensetzung zur Verwendung nach Anspruch 5, wobei die mitochondriale Immuninsuffizienz bei älteren Menschen durch Immunseneszenz verursacht wird.

7. Zusammensetzung zur Verwendung nach einem der Ansprüche 1 bis 6, wobei die Regulierung der Proliferation, der Aktivität und des Überlebens von Immunzellen durch Erhöhung der Produktion von Zytokinen erreicht wird.

8. Zusammensetzung zur Verwendung nach Anspruch 7, wobei das Zytokin IFN-γ und/oder IL-17 ist.

## Revendications

1. Composition comprenant une quantité thérapeutiquement efficace à la fois d'un composé uridine et d'un composé pyruvate, conjointement avec un ingrédient pharmaceutiquement acceptable, pour une utilisation dans une méthode de régulation de la prolifération, de l'activité et de la survie de cellules immunitaires afin d'améliorer et/ou de prévenir les déficits immunitaires causés par une respiration cellulaire altérée.
dans laquelle le composé pyruvate est un sel calcique de pyruvate et le composé uridine est un sel disodique d'uridine-5'-monophosphate.

2. Composition pour l'utilisation selon la revendication 1, dans laquelle les composés uridine et pyruvate sont sous une forme galénique unitaire à administrer simultanément.

3. Composition pour l'utilisation selon la revendication 1 ou 2, dans laquelle elle est présentée sous la forme d'un complément alimentaire ou nutritionnel.

4. Composition pour l'utilisation selon l'une quelconque des revendications 1 à 3, dans laquelle les cellules immunitaires sont des lymphocytes T ou des lymphocytes B.

5. Composition pour l'utilisation selon l'une quelconque des revendications 1 à 4, dans laquelle l'immunodéficience causée par une respiration cellulaire altérée est une immunodéficience choisie parmi une immunodéfìcience mitochondriale primaire, secondaire ou acquise.

6. Composition pour l'utilisation selon la revendication 5, dans laquelle l'immunodéficience mitochondriale est causée par l'immunosénescence chez les personnes âgées.

7. Composition pour l'utilisation selon l'une quelconque des revendications 1 à 6, dans laquelle la régulation de la prolifération, de l'activité et de la survie des cellules immunitaires est obtenue par augmentation de la production de cytokines.

8. Composition pour l'utilisation selon la revendication 7, dans laquelle la cytokine est l'IFN-γ et/ou l'IL-17.
